(19) Europäisches Patentamt
European Patent Office
Office européen des brevets

(11) EP 4 147 702 A1

(12) # EUROPEAN PATENT APPLICATION

(43) Date of publication:
**15.03.2023 Bulletin 2023/11**

(21) Application number: **21196313.7**

(22) Date of filing: **13.09.2021**

(51) International Patent Classification (IPC):
***A61K 31/727*** *(2006.01)* ***A61K 38/48*** *(2006.01)*
***A61P 31/14*** *(2006.01)*

(52) Cooperative Patent Classification (CPC):
(C-Sets available)
**A61P 31/14; A61K 31/727; A61K 38/4813; A61K 45/06; C12Y 304/15001;** C12N 2501/91

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Medizinische Universität Graz**
**8036 Graz (AT)**

(72) Inventors:
- **ZATLOUKAL, Kurt**
  **8036 Graz (AT)**
- **FÖDERL-HÖBENREICH, Esther**
  **8036 Graz (AT)**
- **RIEGER, Julia**
  **8036 Graz (AT)**

(74) Representative: **Loidl, Manuela Bettina et al**
**REDL Life Science Patent Attorneys**
**Donau-City-Straße 11**
**1220 Wien (AT)**

Remarks:
The complete document including Reference Table(s) and the Sequence Listing(s) can be downloaded from the EPO website

(54) **NOVEL COMBINATION OF HEPARIN AND ACE2 DECOYS FOR THE TREATMENT OF COVID-19**

(57) The present invention refers to a combination of heparin, and a recombinant soluble angiotensin-converting enzyme 2 (rsACE2), being employed together such that a synergistic antiviral effect is achieved, and a pharmaceutical preparation containing heparin and rsACE2, specifically for prophylactic or therapeutic treatment of a disease condition which is caused by or associated with an infection by a coronavirus.

EP 4 147 702 A1

(52) Cooperative Patent Classification (CPC): (Cont.)

C-Sets
**A61K 31/727, A61K 2300/00;**
**A61K 38/4813, A61K 2300/00**

**Description**

FIELD OF THE INVENTION

The present invention refers to a combination of heparin, and a recombinant soluble angiotensin-converting enzyme 2 (rsACE2), being employed together such that a synergistic antiviral effect is achieved, and a pharmaceutical preparation containing heparin and rsACE2, specifically for use in the prophylactic or therapeutic treatment of a disease condition caused by or associated with an infection by a coronavirus.

BACKGROUND OF THE INVENTION

The emergence of newly identified viruses highlights the need for the development of novel antiviral strategies. Severe acute respiratory syndrome coronavirus 2 (SARS-CoV-2) is a newly-emerged coronavirus which causes a severe acute respiratory disease (SARS), COVID-19. SARS-CoV-2 was first identified from an outbreak in Wuhan, China in December 2019.

As of August 23, 2021, the World Health Organization has reported 213 million confirmed cases worldwide, resulting in 4.45 million deaths.

SARS-CoV is an animal virus, perhaps bats are the reservoir of the virus, which spread to other animals as well as humans. The transmission of SARS-CoV is primarily from human to human. Transmission of SARS-CoV-2 typically is mediated by aerosols or droplets infecting cells in the nose or pharynx resulting in replication of the virus, which eventually infect cells in the lower respiratory tract resulting in severe respiratory disease (Hoffmann M. et al., 2020).

Moreover, SARS-CoV-2 can also enter the nervous system by crossing the neural-mucosal interface in olfactory mucosa, exploiting the close vicinity of olfactory mucosal, endothelial and nervous tissue, including delicate olfactory and sensory nerve endings.

Symptoms of COVID-19 can range from asymptomatic to mild-illness to pneumonia, renal dysfunction, respiratory and multi-organ failure. Unlike the previous SARS-CoV, COVID-19 has proved to be more infectious. Patients infected with COVID-19 rely on their natural immunity and generally seek supportive care to help relieve symptoms. The major socio-economic threat of the COVID-19 pandemic is caused by cases with severe illness requiring hospitalization and eventually intensive care treatment. Severe COVID-19 results from progression of local infection in the upper respiratory tract to pneumonia. Progression to pneumonia leads to aggravation of symptoms and dyspnea and reduced oxygenation of the blood, which makes treatment in hospitals necessary. At the early stage of pneumonia, virus replication in the lung is the main pathophysiological mechanism causing diffuse alveolar damage, whereas at later stage deregulation of the immune and coagulation systems are the dominating pathophysiological mechanisms.

SARS-CoV-2 is a coronavirus which has four major structural proteins, specifically the surface-anchored trimeric spike (S), envelope (E), membrane (M) and nucleocapsid (N) proteins. The N protein binds to the RNA genome, while the other three structural proteins are components of the viral envelope. The S protein is responsible for allowing the virus to attach and fuse to the membrane of a host cell. SARS-CoV-2 enters a host cell by interacting with the ACE2 receptor on the respiratory epithelial cell using its S protein. The binding results in the activation of several proteases and to the eventual cleavage of the spike protein, which finally leads to fusion of the virus envelope and cell membrane leading to cell infection. The SARS-CoV-2 spike surface-protein is a 1273 amino acid glycoprotein, which can be further subdivided into S1 domain, containing the receptor binding domain (RBD) including the receptor binding motif, and S2 domain. Viral attachment to the ACE2 receptor is implemented through binding of the spike RBD to specific extracellular peptidase domain of ACE2 with a dissociation constant of about Kd ~15nM. Upon interaction, the S2 domain is exposed to proteolytic cleavage which is crucial for subsequent fusion of the viral envelope with the cell membrane of the target cell. As has been already shown for other viruses - such as influenza, herpes simplex but also SARS-CoV-2 related like SARS-CoV and MERS-CoV - the cell-surrounding glycocalyx is often used as a means to gain access to host cells. Moreover, this complex net-like coat of glycoproteins is in fact used by certain pathogens to facilitate cell entry and infection. Many viruses are able to interact with negatively charged cell-surface polysaccharides, the so-called glycosaminoglycans (GAGs), among them is the ubiquitously located heparan sulfate (HS). HS can be divided into subunits of disaccharides, consisting of beta-D-glucuronic acid and alpha-D-glucosamine building blocks, which are massively modified by various sulfations and acetylations, giving them a substantially negative charge. Among all GAGs, HS exhibits the largest amount of heterogeneity, in both, a tissue as well as an individual specific manner, as it undergoes extensive modification during and after synthesis in the Golgi apparatus. In nature, HS exerts most of its functions when covalently bound to a core protein, forming the so-called HS-proteoglycans (HSPGs), which, due to their distinct GAG-pattern, vary tremendously in protein-binding specificity. In addition to their manifold biologic functions, HSPGs, such as the membrane-bound glypicans and syndecans, are often used as an initial anchor point for many pathogens including viruses, which enables the subsequent interaction of viral proteins with their receptors on the host cell, making them co-receptors for viral cell infection.

Specifically for SARS-CoV-2, binding to the ACE2 receptor is strongly dependent upon the interaction between the virus-surface spike protein and cell-surface HSPGs. Binding of the trimeric S protein to HS followed by conformational changes, enhances its binding to ACE2, rendering virus-receptor interaction and subsequent infection more efficient. Molecular modelling but also experimental binding studies have revealed an HS binding site of the spike glycoprotein within its S1 domain, probably in proximity of, but separate from the ACE2 binding domain of S protein, as it binds HS as well as ACE2 in a cooperative manner.

ACE2 is a zinc-containing metalloenzyme located on the surface of intestinal enterocytes, renal tubular cells and other cells. It contains an N-terminal peptidase M2 domain and a C-terminal collectrin renal amino acid transporter domain. ACE2 is a single-pass type I membrane protein, its enzymatically active domain being exposed on the surface of cells in the intestines and other tissues. The extracellular domain of ACE2 is cleaved from the transmembrane domain by a sheddase, and the resulting soluble protein is released into the bloodstream and ultimately excreted in the urine. Both the full-length and soluble ACE2 proteins have the receptor site for the S protein which resides in the extracellular domain.

Soluble ACE2 proteins have already been developed and tested in Phase 1 and 2 clinical trials to prevent SARS-CoV-2 binding to ACE2 (https://clinicaltrials.gov/ct2/show/NCT04335136). These drugs mimick the cellular ACE2 (ACE2 decoys) and bind to the spike protein thereby preventing the virus to bind to and infect human cells. This approach, however, has not demonstrated high efficacy in clinical studies.

Heparin is a close molecular relative of heparan sulfate consisting of the same repeating di-saccharide building blocks but exhibiting pan-sulfation of all available sites. Heparin therefore shows the largest overall negative charge (of all molecules in the body) but in contrast to its GAG family members, it plays little to no role in ECM-enabled signaling and biology since it is solely produced and stored in mast cells. Heparin gathered its attention due to its strong anticoagulant effects by interfering with various steps in the blood coagulation cascade. In addition, heparin possesses anti-inflammatory, immunomodulatory, anti-viral and anti-complement activity which refers to medical benefits beyond anticoagulation. In contrast to unfractionated heparin (UFH), low molecular weight heparin (LMWH) exerts its function only by interfering with the last step of the coagulation cascade, namely the conversion of fibrinogen to fibrin through thrombin. LMWH blocks factor Xa and by this means inhibits the activation of thrombin out of prothrombin thereby preventing blood coagulation. Heparin therapy may reduce the binding of viral spike proteins to HS proteoglycans on the cell surface, thus inhibiting the initial infection or the spread from infected cells to uninfected cells (Liu, J., et al., 2020.).

None of the single compounds, however, have shown to sufficiently inhibit SARS-CoV-2 viral entry for potential used as therapy.

In view of the continuing threat to human health, there is an urgent need for preventive and therapeutic antiviral therapies for SARS-CoV-2 control. Non-vaccine therapies can help to slow down spreading of COVID-19 (antiviral drugs) or ameliorate the consequences of respiratory diseases, thereby minimizing the challenges to health systems and reducing the socio-economic damage. In particular, there is an urgent need for medicines that prevent virus replication, particularly when the disease progresses from local disease in the upper respiratory tract to pneumonia.

## SUMMARY OF THE INVENTION

The objective is solved by the subject of the present claims and as further described herein.

The present invention provides a combination of

(a) heparin, and
(b) a recombinant soluble angiotensin-converting enzyme 2 (rsACE2),

being employed together such that a synergistic antiviral effect is achieved.

According to an embodiment, rsACE2 is an rsACE2 conjugated or fused to a fragment crystallizable (Fc), either directly or via a linker.

According to a further embodiment, the rsACE2 comprises one or more engineered glycans.

In yet a further embodiment, the rsACE2 comprises one or more amino acid substitutions, specifically selected from the group consisting of T27, D30, D31, K31, H34, E35, N90, T92, N322 with reference to SEQ ID NO:1, or any combinations thereof.

According to a specific embodiment, heparin is low molecular heparin, specifically it is any one of enoxaparin, dalteparin and tinzaparin, or any combination thereof.

According to a further embodiment, the synergistic antiviral effect of the combination described herein is against a β-coronavirus whose spike proteins bind to ACE2 receptors, specifically selected from the group consisting of SARS-CoV-2, SARS-CoV-1, HCoV-NL63, and mutants or variants thereof.

Specifically, the synergistic antiviral effect is in reducing infection of susceptible cells by the virus, thereby preventing or treating a disease condition.

According to a further embodiment, one or more further active substances, selected from the group consisting of antiviral, anti-inflammatory and antibiotic substances, such as, but not limited to dexamethasone are added to the combination or are formulated in the pharmaceutical preparation comprising the combination described herein.

The combination described herein can be formulated for local administration, preferably for application to the upper and lower respiratory tract, nasal, nasopharyngeal, pulmonary, intraoral, ocular, or dermal use, or for systemic administration, preferably for parenteral administration, more preferably for inhalation.

Specifically, heparin and rsACE2, either as single components or in a combination preparation, are in the form of a spray, a powder, a liquid solution, a gargle solution, an aerosolized powder, or an aerosolized liquid formulation.

In a further embodiment herein provided is a pharmaceutical preparation comprising the combination described herein together with at least one pharmaceutical carrier.

Specifically, the combination, or the pharmaceutical preparation described herein, is for use in prophylactic or therapeutic treatment of a disease condition which is caused by or associated with an infection by a coronavirus.

Specifically, the disease condition is common cold, infection of the nose, throat and larynx, sinusitis, bronchiolitis, diarrhea, rash on skin, pneumonia, or acute respiratory distress syndrome (ARDS), symptoms of the central nervous system, acute or chronic liver disease, e.g., hepatic steatosis, portal fibrosis, occurrence of lymphocytic infiltrates, ductular proliferation, lobular cholestasis, acute liver cell necrosis, central vein thrombosis; renal proximal tubular injury, focal pancreatitis, adrenocortical hyperplasia, and lymphocyte depletion of spleen and lymph nodes, lung injury such as but not limited to alveolar damage specifically characterized by edema, hyaline membranes, proliferation of pneumocytes and fibroblasts, pulmonary fibrosis, endothelial damage and thrombosis.

Specifically, the combination described herein is administered simultaneously or sequentially, specifically within a sufficiently short interval to ensure that the synergistic antiviral effect is achieved.

According to a further embodiment, provided herein is also the use of the herein described combination in the manufacture of a medicament for prophylactic or therapeutic treatment of a disease condition which is caused by or associated with an infection by a coronavirus.

## FIGURES

Figure 1: Effect of substances on infection of VeroE6 cells. SARS-CoV-2 was pre-treated with Heparin (concentrations: 200μg/mL and 500μg/mL) and ACE2 (concentration: 100μg/mL) as single substances or in combination to infect VeroE6 cells. Virus pre-treated with cell culture medium without substances served as positive control. Virus copy numbers released into the cell culture medium were measured at 24 hrs after infection. Dots show measured virus copy numbers for each experimental series and each replicate. Statistical analysis: ns, not significant; P=0.001 to 0.01 (**).

## DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017), and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, *e.g.*, specifically referring to isolated nucleic acid sequences, amino acid sequences, fusion constructs, expression constructs, transformed host cells and modified proteins, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges as well as by their polarity:

The "neutral" amino acids are shown below along with their respective three-letter and single-letter code and polarity: Alanine (Ala, A; nonpolar, neutral), Asparagine (Asn, N; polar, neutral), Cysteine (Cys, C; nonpolar, neutral),

Glutamine (Gin, Q; polar, neutral), Glycine (Gly, G; nonpolar, neutral), Isoleucine (lie, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).

The "positively" charged amino acids are: Arginine (Arg, R; polar, positive), and Lysine (Lys, K; polar, positive).

The "negatively" charged amino acids are: Aspartic acid (Asp, D; polar, negative), and Glutamic acid (Glu, E; polar, negative).

Heparin, also known as unfractionated heparin, is a medication and naturally occurring glycosaminoglycan. It is well known to act as anticoagulant. Heparin has the structure:

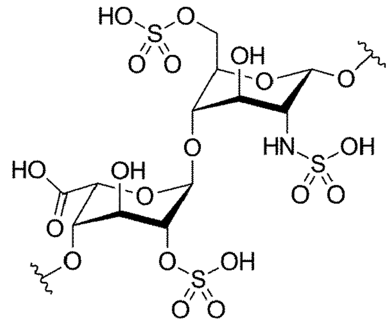

According to the present invention, the term "heparin" refers to unfractionated heparin and selected defined heparin fractions such as LMWH and pharmaceutically acceptable salts thereof. Heparin fractions from about 4.0 to 9.5 kDa can be prepared by depolymerization methods known in the art. Low molecular weight heparins (LMWHs) have a molecular weight of about 4.5 to 6 kDa. LMWHs may be produced by hydrolytic cleavage, chemoenzymatically from disaccharides, and isolated by a variety of techniques, including gel- and ultra-filtration, solvent extraction, and enzymatic or thermal depolymerization. Low molecular heparins useful for the present invention are enoxaparin, dalteparin (sodium), nadroparin (calcium), tinzaparin, ardeparin, bemiparin (sodium), certoparin, 6-O-desulfated heparin, 6-O-desulfated enoxaparin and parnaparin.

The term heparin also refers to heparin analogues or heparinoids such as pentosan polysulfate (PPS), a semi-synthetic polysulfated xylan:

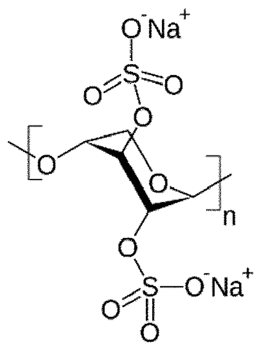

The term heparin analogues further includes low molecular weight heparan sulfate mimetics such as, but not limited to PI-88 (muparfostat or "phosphomannopentaose sulfate", Parish et al. 1999), which is a mixture of highly sulfated mannose-containing di- to hexa-saccharides

The term heparin analogues further includes size defined heparin oligosaccharides. Specifically, heparin fractions are in the range of dp2 (disaccharide) to dp10, specifically the fractions are larger than dp2 and smaller than dp10. Specifically said oligosaccharide fractions have a size of dp4, dp6, or dp8.

Such low molecular weight heparin fractions can be produced by further depolymerisation by partial digestion with heparinase. In particular, partial digestion is performed with heparinase I (Flavobacterium heparinum; heparin lyase EC 4.2.2.7) in 50mM sodium acetate, 0.5mM calcium acetate, pH 7.2 at 37°C (digestion is periodically monitored by rapid gel filtration HPLC of small aliquots of the digest). The final digest is resolved into its constituent oligosaccharide size fractions by gel filtration chromatography.

Pharmaceutically acceptable salts that may be mentioned include acid addition salts and base addition salts. Such salts may be formed by conventional means, for example by reaction of a free acid or a free base form of heparin with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counterion of heparin in the form of a salt with another counter-ion, for example using a suitable ion exchange resin. Examples of pharmaceutically acceptable salts include acid addition salts derived from mineral acids and organic acids, and salts derived from metals such as sodium, magnesium, or particularly, potassium and calcium. Examples of acid addition salts include acid addition salts formed with acetic, 2,2- dichloroacetic, adipic, alginic, aryl sulphonic acids (e.g. benzenesulphonic, naphthalene-2-sulphonic, naphthalene-1 ,5-disulphonic and p-toluenesulphonic), ascorbic (e.g. L-ascorbic), L-aspartic, benzoic, 4-acetamidobenzoic, butanoic, (+) camphoric, camphor-sulphonic, (+)-(1 S)-camphor-10-sulphonic, capric, caproic, caprylic, cinnamic, citric, cyclamic, dodecylsulphuric, ethane-1 ,2-disulphonic, ethanesulphonic, 2-hydroxyethanesulphonic, formic, fumaric, galactaric, gentisic, glucoheptonic, gluconic (e.g. D-gluconic), glucuronic (e.g. D-glucuronic), glutamic (e.g. L-glutamic), a-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulphonic, 1 -hydroxys-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulphuric, tannic, tartaric (e.g.(+)-L-tartaric), thiocyanic, undecylenic and valeric acids.

Recombinant soluble angiotensin-converting enzyme 2 (rsACE2) is an enzyme acting as a carboxypeptidase and cleaving a single residue from AngI, thereby generating AngI-9, and a single residue from AngII to generate AngI-7. Recombinant ACE2 (rACE2), methods of production of ACE2 are described in Crackower et al., 2002, WO 00/18899 A2, WO 02/12471 A2 or WO 2004/000367 A1.

Specifically, human rsACE2 (ACE2) is used in the combination as described herein.

The amino acid sequence of human rsACE2 can be as follows or has at least 90 % sequence identity, specifically at least 95%, more specifically 99% sequence identity with SEQ ID NO:1 or the ACE2 isoform described below.:

MSSSSWLLLSLVAVTAAQSTIEEQAKTFLDKFNHEAEDLFYQSSLASWNYNTNITEENVQ
NMNNAGDKWSAFLKEQSTLAQMYPLQEIQNLTVKLQLQALQQNGSSVLSEDKSKRLNTIL
NTMSTIYSTGKVCNPDNPQECLLLEPGLNEIMANSLDYNERLWAWESWRSEVGKQLRPLY
EEYVVLKNEMARANHYEDYGDYWRGDYEVNGVDGYDYSRGQLIEDVEHTFEEIKPLYEHL
HAYVRAKLMNAYPSYISPIGCLPAHLLGDMWGRFWTNLYSLTVPFGQKPNIDVTDAMVDQ
AWDAQRIFKEAEKFFVSVGLPNMTQGFWENSMLTDPGNVQKAVCHPTAWDLGKGDFRILM
CTKVTMDDFLTAHHEMGHIQYDMAYAAQPFLLRNGANEGFHEAVGEIMSLSAATPKHLKS
IGLLSPDFQEDNETEINFLLKQALTIVGTLPFTYMLEKWRWMVFKGEIPKDQWMKKWWEM
KREIVGVVEPVPHDETYCDPASLFHVSNDYSFIRYYTRTLYQFQFQEALCQAAKHEGPLH
KCDISNSTEAGQKLFNMLRLGKSEPWTLALENVVGAKNMNVRPLLNYFEPLFTWLKDQNK
NSFVGWSTDWSPYADQSIKVRISLKSALGDKAYEWNDNEMYLFRSSVAYAMRQYFLKVKN
QMILFGEEDVRVANLKPRISFNFFVTAPKNVSDIIPRTEVEKAIRMSRSRINDAFRLNDN
SLEFLGIQPTLGPPNQPPVSIWLIVFGVVMGVIVVGIVILIFTGIRDRKKKNKARSGENP
YASIDISKGENNPGFQNTDDVQTSF

(SEQ ID NO:1)

Herein encompassed is also an ACE2 isoform comprising a short ACE2 transcript wherein sequence AWDLGKGDFRILM of SEQ ID NO:1 (SEQ ID NO:2) is replaced by MREAGWDKGGRILM (SEQ ID NO:3, Blume C. et al, 2021) and lacks SARS-CoV-2-spike-high-affinity binding sites.

rsACE2 can form dimers. According to a specific embodiment, also trimeric rsACE2 variants are encompassed herein, e.g. as described by Xiao et al., 2021.

According to a specific embodiment, the ACE2 protein is fused to an IgG Fc-domain, specifically amino acids 18 to 615 of human ACE2 are fused to the Fc-domain of IgG1.

The term "Fc" (or fragment crystallizable region) as used herein refers to the polypeptide comprising the constant region of an antibody excluding the first constant region immunoglobulin domain (CH1 domain) and in some cases, part of the hinge. The Fc region refers to the C- terminal region of an antibody. The Fc region is composed of two identical protein fragments, derived from the second and third constant domains of the antibody's two heavy chains: Chain A and Chain B. The second and third constant domains are known as the CH2 domain and the CH3 domain, respectively. The CH2 domain comprises a CH2 domain sequence of Chain A and a CH2 domain sequence of Chain B. The CH3 domain comprises a CH3 domain sequence of Chain A and a CH3 domain sequence of Chain B. As used herein, the Fc region includes the hinge region or a part thereof.

By "fused" or "connected" is meant that the components (e.g. rsACE2 and an Fc domain subunit) are linked by peptide bonds, either directly or via one or more peptide linkers.

The term "linker" as used herein refers to a peptide linker and is preferably a peptide with an amino acid sequence with a length of 2, 3, 4, 5, 6, 7 or more amino acids, preferably with a length of 2-10, more preferably of 3-5 amino acids.

The Fc may include the hinge region or a part thereof.

ACE2 Fc can be expressed by any host cell appropriate for protein expression, such as bacterial, plant or animal cells, e.g. HEK293 cells, Vero cells, CHO cells or plant cells such as *Nicotiana benthamiana.* More specifically, expression is performed in *N. benthamiana* ΔXT/FT cells. ACE2 may further be enzymatically deglycosylated, e.g. with endoglycosidase H or peptide-N-glycosidase F as described in Castilho A. et al., 2021.

According to a further embodiment described herein, ACE2 comprises one or more amino acid modifications, deletions or substitutions, specifically selected from the group consisting of T27, D30, K31, H34, E35, L79, N90, T92, N322, N330 with reference to SEQ ID NO:1 (Chan K.K. et al., 2020) and any combinations thereof. ACE2 glycosylation motifs are located at positions N90 and N322. Specifically, the ACE2 is ACE2-T92Q-Fc, ACE2-N322Q, or ACE2-T92Q-N322Q-Fc or an ACE2 lacking the Fc domain. Specifically, the rsACE2 may also be an enzymatically desialylated ACE2 or ACE2-Fc as described by Capraz T et al., 2021.

In accordance with the present invention, components (a) and (b) of the combination may be administered simultaneously or sequentially. In the latter case, however, the components are administered within a sufficiently short interval to ensure that a synergistic antiviral effect is achieved.

The term "synergistic antiviral effect" is used to denote an antiviral effect which is greater than the predicted purely additive effects of the individual components (a) and (b) of the combination.

An advantage of the combination of the present invention is that it enables attainment of an improved antiviral efficacy at a particular dose of one of the antiviral components, i.e. heparin and rsACE2, (compared with heparin or rsACE2 used alone) thereby improving the therapeutic index of the component. Thus, for example, the combination may be used to treat conditions which would otherwise require relatively large doses of the antiviral component at which toxicity problems may occur and makes therapy with recombinat proteins very expensive thereby exluding many patients benfitting from such therapies The smaller doses of the combination may provide increased convenience to the patient and increased compliance.

While it is possible for the active components heparin and rsACE2 to be administered alone, it is preferable to present them as a pharmaceutical preparation. The pharmaceutical preparation of the present invention comprises a combination described herein, together with one or more acceptable carriers and optionally further therapeutic agents. Each carrier must be "acceptable" in the sense of being compatible with the other ingredients of the formulation and not injurious to the patient. Formulations include those suitable for oral, nasal, nasopharyngeal, inhalation, or topical (including buccal and sublingual) administration or for lung lavage.

According to one embodiment, the combination or pharmaceutical preparation described herein is formulated for local administration, such as for application to the upper and lower respiratory tract, intranasal, pulmonary inhalation or lung lavage.

For administration by inhalation, heparin can be delivered in the form of an aerosol spray from pressured container or dispenser which contains a suitable propellant, e.g., a gas such as carbon dioxide, or a nebulizer. As used herein, the term "aerosols" refers to dispersions in air of solid particles, of fine enough particle size and consequent low settling velocities to have relative airborne stability. The nebulization of heparin may be achieved by a gas pressure or by ultrasound.

Generally speaking, a nebulizer is an apparatus permitting the administration of aerosols. The nebulizers such as aerosol and ultrasonic nebulizers may be of any type and their structures are known to a person skilled in the art, and these devices are commercially available. The aerosols of the invention can be made by nebulizing a heparin containing preparation or combination described herein using a variety of known nebulizing techniques.

There are a variety of nebulizers that are available to produce aerosols including small volume nebulizers. Compressor driven nebulizers incorporate jet technology and use compressed air or medical oxygen to generate the aerosol.

Ultrasonic nebulizers, e.g., an ultrasonic type nebulizer with a quartz crystal vibrating at high frequency, can also be used to deliver the heparin preparation.

Dry powder inhalation systems can also be used, which may be passive or active delivery devices.

The term "effective" or "effective amount" with respect to an antiviral effect as used herein, shall refer to an amount (in particular a predetermined amount) that has a proven antiviral effect. The amount is typically a quantity or activity sufficient to, when administered to a subject, effect beneficial of desired antiviral results, and, as such, an effective amount or synonym thereof depends upon the context in which it is being applied.

An effective amount of a pharmaceutical preparation or drug is intended to mean that amount of heparin/rsACE2 that is sufficient to treat, prevent or inhibit a disease, disease condition or disorder. Such an effective dose specifically

refers to that amount of the compound sufficient to result in healing, prevention or amelioration of conditions related to diseases or disorders described herein.

The concentration of heparin specifically is in the range of 50 to 1000 μg/mL, the concentration of ACE2 is in the range of 5 to 250 μg/mL, specifically in the range of a10 to 200 μg/mL.

Specifically, the present disclosure also provides a nasal administration formulation of heparin and rsACE2. Said nasal administration formulation may further contain sodium chloride and water for injection; the concentration of the heparin in the nasal administration formulation specifically is in the range of 50 to 1000 μg/mL, the concentration of ACE2 is in the range of 5 to 250 μg/mL, specifically in the range of a10 to 200 μg/mL.

The nasal or inhalation administration agent of the combination provided in the present disclosure avoids bleeding and other adverse effects that are likely to occur in subcutaneous injection. Moreover, nasal and inhalation administration are very safe. When heparin and ACE2 are administrated through the respiratory tract, the level of drug in the blood would not exceed the safe level even at a high drug concentration.

The formulations may conveniently be presented in unit dosage form and may be prepared by any methods well known in the art of pharmacy. Such methods include the step of bringing into association the active ingredients with the carrier which constitutes one or more accessory ingredients.

The term "antiviral" as used herein shall refer to any substance, drug or preparation, that effects the biology of a virus and attenuates or inhibits viral attachment, entry, replication, shedding, latency or a combination thereof, resulting in reduction of viral load or infectivity.

The terms "attenuating", "inhibiting", "reducing", or "preventing", or any variation of these terms, when used in the claims and/or the specification includes any measurable decrease or complete inhibition to achieve a desired result, e.g., reduction in the risk of viral infection (pre-exposure), or reduction of post-exposure viral survival, viral load, or virus growth.

The term "susceptible cells" refers to cells serving as portal of virus entry, e.g. cells expressing ACE2, specifically, said cells can be, but are not limited to respiratory cells, epithelial cells, enterocytes, or neural cells.

A treatment or prevention regime of a subject with an effective amount of spray dried heparin described herein may consist of a single application or administration, or alternatively comprise a series of applications and administrations, respectively. For example, the combination described herein may be used at least once a month, or at least once a week, or at least once a day. However, in certain cases of an acute phase, e.g. upon suspected or confirmed exposure to a virus, or after virus infection has been determined, the spray dried heparin in combination with the rsACE2 described herein may be used more frequently, e.g. 1-10 times a day.

Specifically, a combination therapy is provided which includes treatment with the preparation described herein and standard therapy of a coronavirus-caused disease.

Doses may be applied in combination with other active agents such as antiviral agents, anti-inflammatory drugs or antibiotics, e.g. upon the subject's risk of viral spread, so to prevent a pathogen associated reaction.

Treatment can be combined with an antiviral, anti-inflammatory or antibiotic treatment, preferably wherein a pharmaceutical preparation is administered before, during (e.g., by co-administration or in parallel), or after said antiviral, anti-inflammatory or antibiotic treatment.

The length of the treatment period depends on a variety of factors, such as the severity of the disease, either acute or chronic disease, the age of the patient, and the concentration of the respective components of the combination described herein. It will also be appreciated that the effective dosage used for the treatment or prophylaxis may increase or decrease over the course of a particular treatment or prophylaxis regime. Changes in dosage may result and become apparent by standard diagnostic assays known in the art.

The pharmaceutical preparation or medicinal product described herein is specifically provided as human or veterinary pharmaceutical composition or medicinal product. Medicinal products are understood as substances that are used to treat diseases, to relieve complaints, or to prevent such diseases or complaints in the first place. This definition applies regardless of whether the medicinal product is administered to humans or to animals. The substances can act both within or on the body.

Specifically, the combination described herein is administered to a subject at an early stage of the coronavirus infection. By "early stage" of the coronavirus infection signs and symptoms of COVID-19 may appear 2 to 14 days after virus exposure. This time after exposure and before having symptoms is called the incubation period. At early stage, the subject is tested positive, but has only mild to moderate symptoms. Common signs and symptoms can include fever, cough, tiredness. Early stage symptoms of COVID-19 may also include a loss of taste or smell. Early stage may further refer to a stage with onset of pneumonia and minor impairment of blood oxygenation in COVID-19 patients (Tian W. et al., 2020).

A "pharmaceutically acceptable carrier" refers to an ingredient in a formulation for medicinal or medical use, other than an active ingredient, which is nontoxic to a subject. A pharmaceutically acceptable carrier includes, but is not limited to, a buffer, excipient, stabilizer, or preservative, and the like. Preferably, the buffer has a pH in the range of 6 to 10, specifically it has a pH of 6, 6.5, 7, 7.5, 8, 8.5, 9, 9.5, 10. Additional pharmaceutically acceptable carriers are known

in the art and described in, e.g., Remington: The Science and Practice of Pharmacy, 22nd revised edition (Allen Jr, LV, ed., Pharmaceutical Press, 2012). Liquid formulations can be solutions, emulsions or suspensions and can include excipients such as suspending agents, solubilizers, surfactants, preservatives, and chelating agents.

The term "subject" as used herein shall refer to a warm-blooded mammalian, particularly a human being or a non-human animal, including e.g., dogs, cats, rabbits, horses, cattle, pigs and rodents.

The term "patient" includes human and other mammalian subjects that receive either prophylactic or therapeutic treatment. The term "patient" as used herein always includes subjects without symptoms.

Specifically, the subject is or has been exposed to a coronavirus, or is otherwise at risk of being infected with the coronavirus. Specifically, the subject has been determined or diagnosed of being infected with the virus. In specific embodiments, a subject is treated which is a diseased subject or patient suffering from Coronaviridae virus-caused disease, e.g., a SARS virus-caused disease, upon getting in contact with the pathogen, such as COVID19, or COVID19-associated pneumonia.

In particular the treatment and medical use described herein applies to a subject in need of prophylaxis or therapy of a disease condition associated with a coronavirus infection. Specifically, the treatment may be by interfering with the pathogenesis of a disease condition where a coronavirus is a causal agent of the condition. The subject may be a patient at risk of such disease condition or suffering from disease.

The term "at risk of" a certain disease condition, refers to a subject that potentially develops such a disease condition, e.g. by a certain predisposition, exposure to virus or virus-infected subjects, or that already suffers from such a disease condition at various stages, particularly associated with other causative disease conditions or else conditions or complications following as a consequence of viral infection. The risk determination is particularly important in a subject, where a disease has not yet been diagnosed. This risk determination therefore includes early diagnosis to enable prophylactic therapy. Specifically, the combination is used in subjects with a high risk, e.g. a high probability of developing disease.

The term "treatment" is thus meant to include both prophylactic and therapeutic treatment.

Specifically, the term "prophylaxis" refers to preventive measures which is intended to encompass prevention of the onset of disease or prophylactic measures to reduce the risk of disease progression.

The term "therapy" as used herein with respect to treating subjects refers to medical management of a subject with the intent to cure, ameliorate, stabilize, reduce the incidence or prevent a disease, pathological condition, or disorder, which individually or together are understood as "disease condition". The term includes active treatment, directed specifically toward the improvement of a disease condition, prophylaxis directed specifically toward the prevention of a disease condition, and also includes causal treatment directed toward removal of the cause of the associated disease condition. In addition, this term includes palliative treatment designed for the relief of symptoms rather than the curing of the disease condition, and further curing a disease condition directed to minimizing or partially or completely inhibiting the development of the associated disease condition, and supportive treatment employed to supplement another specific therapy directed toward the improvement of the associated disease condition.

The foregoing description will be more fully understood with reference to the following examples. Such examples are, however, merely representative of methods of practicing one or more embodiments of the present invention and should not be read as limiting the scope of invention.

## EXAMPLES

Example 1:

Increased SARS-COV-2 neutralizing activity of the combination of an ACE2 decoy with heparin

Material & Methods

**Cells and virus**

African green monkey kidney epithelial cells (VeroE6) were purchased from Biomedica (VC-FTV6) and maintained in Minimum Essential Medium (MEM, Thermofisher) containing Earle's Salts and L-Glutamine, supplemented with 5 % heat inactivated fetal calve serum (FCS) and 1 % of penicillin/streptomycin. Human 2019-nCoV Isolate (Ref-SKU: 026V-03883, purchased from Charité, Berlin, Germany) was propagated in VeroE6 cells. SARS-CoV-2 virus titers were determined using a focus forming assay.

ACE2 was kindly provided by Lukas, Mach University of The University of Natural Resources and Applied Life Sciences, Vienna. A low molecular weight heparin was purchased from the pharmacy (Lovonexo®, Enoxaparin-Natrium, 200 mg/mL) and is further referred to as heparin.

**SARS-CoV-2 neutralization assay**

VeroE6 cells were seeded in 48-well cell culture plates ($3 \times 10^4$ cells/well) in MEM supplemented with 2 % FBS overnight to reach approximately 80 % confluence on the day of infection. 100 μg/ml ACE2-wt and 200 μg/ml Heparin were pre-incubated for 30 min at 37 °C under constant shaking (300 rpm) with SARS-CoV-2, either separated or in combination. Mock-treated virus, served as positive control. After preincubation, VeroE6 were infected with MOI = 0.002 for 1 hr, 37 °C, 5 % $CO_2$. Subsequently, cells were washed with MEM to remove unadsorbed virus and were maintained in MEM supplemented with 2 % FBS. After 24 hrs, viral RNA was extracted from the supernatant and quantified via qRT-PCR.

**SARS-CoV-2 RNA isolation and quantification via qRT-PCR**

Viral RNA was extracted from the supernatant using QiaAmp Viral RNA Minikit (QIAGEN, Hilden, Germany), according to the manufacturer's protocol. SARS-CoV-2 replication was quantified via qRT-PCR using N2 primer and probe from 2019-Novel Coronavirus (2019-nCoV) Real-time rRT-qPCR Panel [1] and QuantiTect Multiplex RT-qPCR Kit (Qiagen GmbH, Hilden, Germany) with a Rotor Gene Q cycler (Qiagen GmbH, Hilden, Germany). Amplification was performed in a total volume of 25 μL at 50 °C for 30 min and 95 °C for 15 min followed by 45 cycles of 95 °C for 3 s and 55 °C for 30 s. The primer and probe set for viral RNA quantification are stated below:

2019-nCoV_N2-F 2019-nCoV_N2 Forward Primer 5'- TTA CAA ACA TTG GCC GCA AA-3' (SEQ ID NO:4)
2019-nCoV_N2-R 2019-nCoV_N2 Reverse Primer 5'- GCG CGA CAT TCC GAA GAA -3' (SEQ ID NO:5)
2019-nCoV_N2-P 2019-nCoV_N2 Probe 5'- FAM-ACA ATT TGC CCC CAG CGC TTC AG-BHQ1-3' (SEQ ID NO:6)

A commercially available copy number standard (ATCC VR-1986D genomic RNA from 2019 Novel Coronavirus, Lot: 70035624) was serially diluted and analyzed by qRT-PCR. The resulting $C_q$-values were plotted against In[copy numbers] and the equation obtained from a simple linear regression analysis was used to calculate the copy numbers from the $C_q$-values.

$$y = -1.51 + 38.357$$

Calculated copy numbers refer to 5 μL eluate used as template in qRT-PCR.

Figure 1 shows the effect of substances on infection of VeroE6 cells. SARS-CoV-2 was pre-treated with Heparin (concentrations: 200μg/mL and 500μg/mL) and ACE2 (concentration: 100μg/mL) as single substances or in combination to infect VeroE6 cells. Virus pre-treated with cell culture medium without substances served as positive control. Virus copy numbers released into the cell culture medium were measured at 24 hrs after infection. Dots show measured virus copy numbers for each experimental series and each replicate. Statistical analysis: ns, not significant; P=0.001 to 0.01 (**).

**Statistical analysis**

Statistical analysis was conducted using GraphPad Prism 9. Significance was determined by Kruskal-Wallis (comparing mean rank of control column (= positive control) with mean rank of every other column). The p-value significance level is defined as follows: P= 0.01 to 0.05 (*); P=0.001 to 0.01 (**).

Results

**Heparin and soluble ACE2 show synergistic antiviral effect against SARS-CoV-2 infection in VeroE6 cells.**

To test, whether ACE2 and heparin show synergistic antiviral effects, the two substances were pre-incubated separately and in combination with the virus and then applied to VeroE6 cells to allow infection. Virus replication was measured by determining virus copy numbers released into the cell culture medium at 24 hours after infection. Figure 1 shows combined results of two independent experiments with three replicas each. Results obtained demonstrate that pre-incubation with heparin at concentrations of 200μg/mL and 500μg/mL had no significant effect on viral replication. Pre-treatment with 100 μg/ml ACE2 resulted in some but not significant reduction in detected virus copy numbers. Pre-treatment with a combination of both substances lead to significantly reduced virus copy numbers in all replicates of both experiments.

REFERENCES

Blume C. et al, 2021, A novel ACE2 isoform is expressed in human respiratory epithelia and is upregulated in response to interferons and RNA respiratory virus infection, Nature Genetics 53:205-214 2021
Capraz T et al, 2021, Structure-guided glyco-engineering of ACE2 for improved potency as soluble SARS-CoV-2 decoy receptor, bioRxiv preprint doi: https://doi.org/10.1101/2021.08.31.458325
Castilho A. et al., Generation of enzymatically competent SARS-CoV-2 decoy receptor ACE2-Fc in glycoengineered Nicotiana benthamiana, Biotechnol.J., 2021, 16, 1-6
Chan K.K. et al., Engineering human ACE2 to optimize binding to the spike protein of SARS coronavirus 2, 2020, Science, 369, 1261-1265
Crackower M.A., et al., Angiotensin-converting enzyme 2 is an essential regulator of heart function, Nature 2002 Jun 20;417(6891):822-8., doi: 10.1038/nature00786
Hoffmann, M., et D al., SARS-CoV-2 Cell Entry Depends on ACE2 and TMPRSS2 and Is Blocked by a Clinically Proven Protease Inhibitor. Cell, 2020. 2(181): p. 271-280)
Liu, J., et al., Using heparin molecules to manage COVID-2019. Res Pract Thromb Haemost, 2020. 4(4): p. 518-523.
Prevention CfDCa. Research Use Only 2019-Novel Coronavirus (2019-nCoV) Real-time RT-PCR Primers and Probes [updated 21/06/0621/02/09]
Tian W. et al., Immune suppression in the early stage of COVID-19 disease, 2020, Nature Communications
Xiao T. et al, 2021, A trimeric human angiotensin-converting enzyme 2 as an antiSARS-CoV-2 agent, NatStruct-MolBiol., 28(2):202-209

## Claims

**1.** A combination of

(a) heparin, and
(b) a recombinant soluble angiotensin-converting enzyme 2 (rsACE2),

being employed together such that a synergistic antiviral effect is achieved.

**2.** The combination of claim 1, wherein the rsACE2 is an rsACE2 fused to a fragment crystallizable (Fc).

**3.** The combination of claim 1 or 2, wherein the rsACE2 comprises one or more engineered glycans.

**4.** The combination of claim 1 to 3, wherein the rsACE2 comprises one or more amino acid substitutions, specifically selected from the group consisting of T27, D30, D31, K31, H34, E35, N90, T92, N322 with reference to SEQ ID NO:1, or any combinations thereof.

**5.** The combination of claim 1, wherein heparin is low molecular heparin, specifically selected from the group consisting of enoxaparin, dalteparin and tinzaparin.

**6.** The combination of any one of claims 1 to 5, wherein the synergistic antiviral effect is against a β-coronavirus, specifically selected from the group consisting of SARS-CoV-2, SARS-CoV-1, HCoV-NL63, and mutants thereof.

**7.** The combination of any one of claims 1 to 6, wherein the synergistic antiviral effect is in reducing infection of susceptible cells by the virus.

**8.** The combination of any one of claims 1 to 7, and one or more further active substances, selected from the group consisting of antiviral, anti-inflammatory and antibiotic substances.

**9.** The combination of any one of claims 1 to 8, formulated for local administration, preferably for application to the upper and lower respiratory tract, nasal, pulmonary, intraoral, ocular, or dermal use, or for systemic administration, preferably for parenteral administration, more preferably for inhalation or lung lavage.

**10.** The combination of any one of claims 1 to 9, wherein (a) and/or (b) are in the form of a spray, a powder, a liquid

solution, a gargle solution, an aerosolized powder, or an aerosolized liquid formulation.

**11.** A pharmaceutical preparation comprising a combination of any one of claims 1 to 10, optionally together with at least one pharmaceutical carrier.

**12.** The combination of any one of claims 1 to 10, or the pharmaceutical preparation of claim 11, for use in prophylactic or therapeutic treatment of a disease condition which is caused by or associated with an infection by a coronavirus.

**13.** The combination or the pharmaceutical preparation for use of claim 12, wherein the disease condition is common cold, infection of the nose, throat and larynx, sinusitis, bronchiolitis, diarrhea, rash on skin, pneumonia, or acute respiratory distress syndrome (ARDS), symptoms of the central nervous system, acute or chronic liver disease, hepatic steatosis, portal fibrosis, occurrence of lymphocytic infiltrates, ductular proliferation, lobular cholestasis, acute liver cell necrosis, central vein thrombosis; renal proximal tubular injury, focal pancreatitis, adrenocortical hyperplasia, lymphocyte depletion of spleen and lymph nodes, lung injury; alveolar damage specifically **characterized by** edema, hyaline membranes, proliferation of pneumocytes and fibroblasts, pulmonary fibrosis, endothelial damage and thrombosis.

**14.** The combination or the pharmaceutical preparation for use of claim 12 or 13, which is administered simultaneously or sequentially, specifically within a sufficiently short interval to ensure that the synergistic antiviral effect is achieved.

**15.** Use of a combination of any one of claims 1 to 10 in the manufacture of a medicament for prophylactic or therapeutic treatment of a disease condition which is caused by or associated with an infection by a coronavirus.

Fig. 1

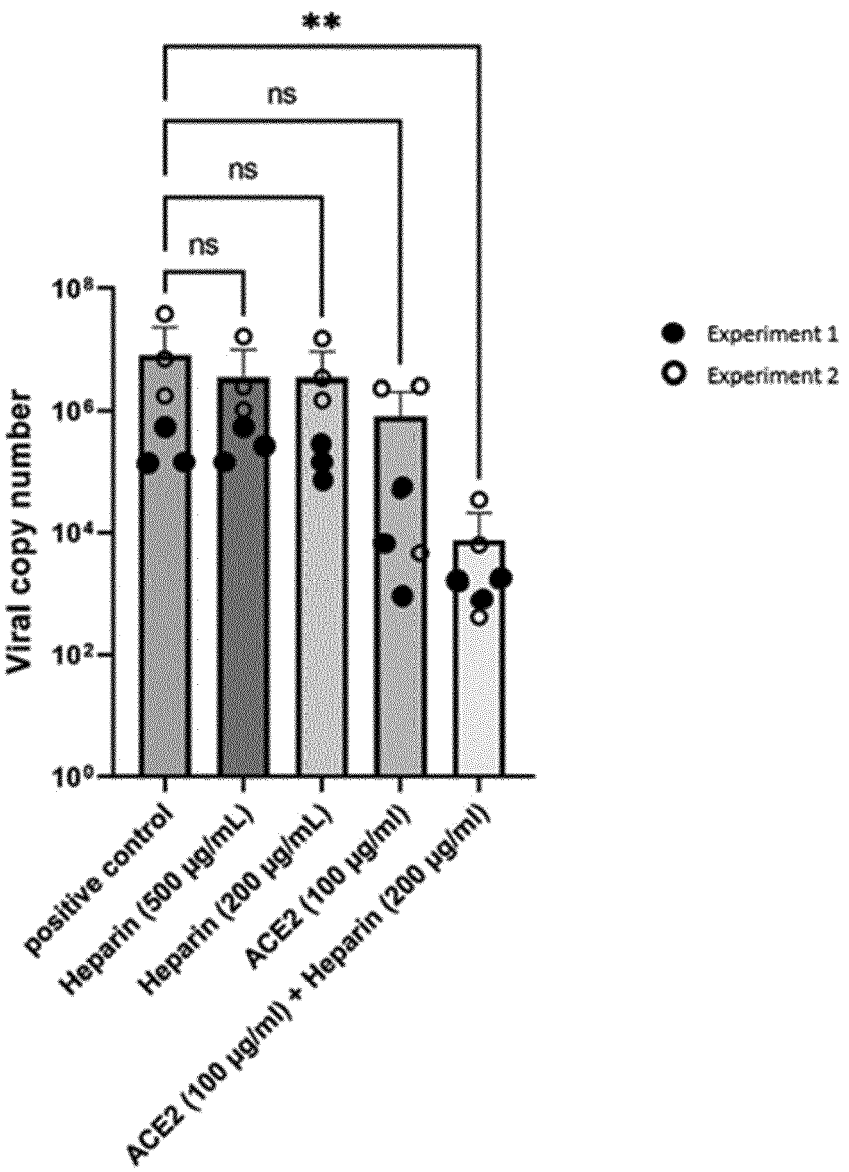
**
ns
ns
ns
Viral copy number
$10^8$
$10^6$
$10^4$
$10^2$
$10^0$
positive control
Heparin (500 µg/mL)
Heparin (200 µg/mL)
ACE2 (100 µg/ml)
ACE2 (100 µg/ml) + Heparin (200 µg/ml)
Experiment 1
Experiment 2

Europäisches Patentamt
European Patent Office
Office européen des brevets

# EUROPEAN SEARCH REPORT

Application Number
EP 21 19 6313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2021/151043 A2 (HAN JANG HYUN [US]; RUTTER BILL [US]; SEO MI YOUNG [KR]) 29 July 2021 (2021-07-29)<br>* abstract *<br>* page 1, paragraph 0003 - page 55, paragraph 0313 *<br>* examples *<br>* claims *<br>----- | 1-15<br>1-15 | INV.<br>A61K31/727<br>A61K38/48<br>A61P31/14 |
| Y | WO 2021/174107 A2 (UNIV NORTHWESTERN [US]) 2 September 2021 (2021-09-02)<br>* abstract *<br>* page 1, paragraph 2 - page 38, paragraph 162 *<br>* examples *<br>* claims *<br>----- | 1-15 | |
| Y | ANUM GLASGOW ET AL: "Engineered ACE2 receptor traps potently neutralize SARS-CoV-2", PROCEEDINGS OF THE NATIONAL ACADEMY OF SCIENCES, 22 October 2020 (2020-10-22), XP055745679, ISSN: 0027-8424, DOI: 10.1073/pnas.2016093117<br>* abstract *<br>* page 1, left-hand column, paragraph 1 - page 9, right-hand column, paragraph 3 *<br>-----<br>-/-- | 1-15 | TECHNICAL FIELDS SEARCHED (IPC)<br>A61K<br>A61P<br>C12N |

2

The present search report has been drawn up for all claims

| Place of search | Date of completion of the search | Examiner |
|---|---|---|
| Munich | 16 February 2022 | Camilleri, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 19 6313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TANAKA SHIHO ET AL: "An ACE2 Triple Decoy that neutralizes SARS-CoV-2 shows enhanced affinity for virus variants", SCIENTIFIC REPORTS, vol. 11, no. 1, 17 June 2021 (2021-06-17), XP055866842, DOI: 10.1038/s41598-021-91809-9 Retrieved from the Internet: URL:http://www.nature.com/articles/s41598-021-91809-9> * abstract * * page 1, paragraph 1 - page 9, paragraph 6 * ----- | 1-15 | |
| Y | Mycroft-West Courtney J. ET AL: "Heparin inhibits cellular invasion by SARS-CoV-2: structural dependence of the interaction of the surface protein (spike) S1 receptor binding domain with heparin.", bioRxiv, 8 May 2020 (2020-05-08), XP055819235, DOI: 10.1101/2020.04.28.066761 Retrieved from the Internet: URL:https://www.biorxiv.org/content/10.1101/2020.04.28.066761v2.full.pdf [retrieved on 2021-06-29] * abstract * * page 2, paragraph 1 - page 9, paragraph 4 * * figures 1-7 * * tables 1-3 * ----- | 1-15 | TECHNICAL FIELDS SEARCHED (IPC) |

2

The present search report has been drawn up for all claims

| Place of search | Date of completion of the search | Examiner |
|---|---|---|
| Munich | 16 February 2022 | Camilleri, Alain |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT ON EUROPEAN PATENT APPLICATION NO.

EP 21 19 6313

This annex lists the patent family members relating to the patent documents cited in the above-mentioned European search report. The members are as contained in the European Patent Office EDP file on
The European Patent Office is in no way liable for these particulars which are merely given for the purpose of information.

16-02-2022

| Patent document cited in search report | Publication date | Patent family member(s) | Publication date |
|---|---|---|---|
| WO 2021151043 A2 | 29-07-2021 | NONE | |
| WO 2021174107 A2 | 02-09-2021 | US 2021371841 A1 | 02-12-2021 |
| | | WO 2021174107 A2 | 02-09-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0018899 A2
- WO 0212471 A2
- WO 2004000367 A1


### Non-patent literature cited in the description


- **SAMBROOK et al.** Molecular Cloning: A Laboratory Manual. Cold Spring Harbor Laboratory Press, 2012, vol. 1 -3
- **KREBS et al.** Lewin's Genes XI. Jones & Bartlett Learning, 2017
- **MURPHY ; WEAVER.** Janeway's Immunobiology. Taylor & Francis Inc, 2017
- Remington: The Science and Practice of Pharmacy. Pharmaceutical Press, 2012
- **BLUME C et al.** A novel ACE2 isoform is expressed in human respiratory epithelia and is upregulated in response to interferons and RNA respiratory virus infection. *Nature Genetics,* 2021, vol. 53, 205-214
- **CAPRAZ T et al.** Structure-guided glyco-engineering of ACE2 for improved potency as soluble SARS-CoV-2 decoy receptor. *bioRxiv,* 2021
- **CASTILHO A et al.** Generation of enzymatically competent SARS-CoV-2 decoy receptor ACE2-Fc in glycoengineered Nicotiana benthamiana. *Biotechnol.J.,* 2021, vol. 16, 1-6
- **CHAN K.K. et al.** Engineering human ACE2 to optimize binding to the spike protein of SARS coronavirus 2. *Science,* 2020, vol. 369, 1261-1265
- **CRACKOWER M.A. et al.** Angiotensin-converting enzyme 2 is an essential regulator of heart function. *Nature,* 20 June 2002, vol. 417 (6891), 822-8
- **HOFFMANN, M.** SARS-CoV-2 Cell Entry Depends on ACE2 and TMPRSS2 and Is Blocked by a Clinically Proven Protease Inhibitor. *Cell,* 2020, vol. 2 (181), 271-280
- **LIU, J. et al.** Using heparin molecules to manage COVID-2019. *Res Pract Thromb Haemost,* 2020, vol. 4 (4), 518-523
- **TIAN W et al.** Immune suppression in the early stage of COVID-19 disease. *Nature Communications,* 2020
- **XIAO T et al.** A trimeric human angiotensin-converting enzyme 2 as an antiSARS-CoV-2 agent. *NatStructMolBiol.,* 2021, vol. 28 (2), 202-209